(19) Europäisches Patentamt / European Patent Office / Office européen des brevets

(11) **EP 4 230 732 B1**

(12) **EUROPEAN PATENT SPECIFICATION**

(45) Date of publication and mention of the grant of the patent:
**22.01.2025  Bulletin 2025/04**

(51) International Patent Classification (IPC):
*C12N 5/10* (2006.01)     *C12N 15/12* (2006.01)
*C12N 15/85* (2006.01)    *C12R 1/91* (2006.01)
*C07K 14/48* (2006.01)    *C12N 5/071* (2010.01)
*G01N 33/74* (2006.01)

(21) Application number: **21951374.4**

(22) Date of filing: **30.07.2021**

(52) Cooperative Patent Classification (CPC):
**C12N 5/0682; C07K 14/48; G01N 33/74;**
C12N 2500/90; C12N 2510/00; C12N 2510/02;
C12N 2740/16043; C12N 2800/22;
G01N 2333/4716; G01N 2333/48

(86) International application number:
**PCT/CN2021/109763**

(87) International publication number:
**WO 2023/004784 (02.02.2023 Gazette 2023/05)**

(54) **RECOMBINANT CHO CELL, CONSTRUCTION METHOD THEREFOR, AND DETECTION SYSTEM AND METHOD USING SAME**

REKOMBINANTE CHO-ZELLE, KONSTRUKTIONSVERFAHREN DAFÜR SOWIE DETEKTIONSSYSTEM UND -VERFAHREN DAMIT

CELLULE CHO RECOMBINANTE, SON PROCÉDÉ DE CONSTRUCTION, ET SYSTÈME DE DÉTECTION ET PROCÉDÉ L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**23.08.2023  Bulletin 2023/34**

(73) Proprietor: **Chengdu Unovel Biopharma Co., Ltd.**
**Chengdu, Sichuan 610000 (CN)**

(72) Inventors:
• **LI, Zhushi**
  **Chengdu, Sichuan 610000 (CN)**
• **CHEN, Hong**
  **Chengdu, Sichuan 610000 (CN)**
• **WANG, Ying**
  **Chengdu, Sichuan 610000 (CN)**

(74) Representative: **Thoma, Michael**
**Lorenz Seidler Gossel**
**Rechtsanwälte Patentanwälte**

**Partnerschaft mbB**
**Widenmayerstraße 23**
**80538 München (DE)**

(56) References cited:
**EP-A1- 0 414 151**      **EP-A1- 3 431 494**
**CN-A- 102 586 190**    **CN-A- 108 034 678**
**CN-A- 108 300 736**    **US-A- 5 082 774**
**US-A- 5 169 762**      **US-A- 5 457 034**

• **WEI YUE, LUJIA PENG;QUANWEI SHI;YANHUA GE;HEZHONG LIU;BEIJING: "Study on the High Level Expression and Bioactivity Evaluation of Recombinant Human Nerve Growth Factor", ZHONGGUO YAOSHI, ZHONGGUO YAOSHI ZAZHI BIANJIBU, CN, vol. 28, no. 6, 20 June 2014 (2014-06-20), CN , pages 601 - 606, XP093030106, ISSN: 1002-7777, DOI: 10.16153/j.1002-7777.2014.06.010**

- **XU, LI ET AL.: "Expression, purification, and characterization of recombinant mouse nerve growth factor in Chinese hamster ovary cells", PROTEIN EXPRESSION AND PURIFICATION, vol. 104, 20 September 2014 (2014-09-20), XP009542988, ISSN: 1046-5928**

Remarks:
    The complete document including Reference Table(s) and the Sequence Listing(s) can be downloaded from the EPO website

Remarks:
    The complete document including Reference Table(s) and the Sequence Listing(s) can be downloaded from the EPO website

**Description**

**TECHNICAL FIELD**

The present application belongs to the field of immune effect detection, and specifically relates to a recombinant CHO cell, a method of constructing the recombinant cell, and a detection system and method for detecting an ADCC effect and a CDC effect of an antibody using the cell.

**BACKGROUND**

ADCC, i.e., antibody-dependent cell-mediated cytotoxicity, refers to that after an antibody specifically binds to antigenic determinants on a surface of a target cell through Fab segment, a Fc segment thereof binds to an effector cell with FcγR, triggering killing activity of the effector cell to directly kill the target cell. An ADCC effect is an important immune mechanism for anti-infection and anti-tumor of the body. The effector cells involved in ADCC mainly include natural killed (NK) cell, phagocyte, eosinophil, basophil and mast cell, and the like.

CDC i.e., complement dependent cytotoxicity, refers to a cytotoxic effect in which a complement is involved, that is, a complex is formed to activate a complement classical pathway through the binding of a specific antibody to the corresponding antigen on the surface of cell membrane. Complement protein C1q initiates the complement classical pathway firstly, and then complement proteins C2-C9 are activated to form a membrane attack complex (MAC), which exerts a lysis effect on the target cell.

In view of the important role of the ADCC effect and CDC effect in tumor immunity, in the current development of anti-tumor antibodies, many researchers expect to obtain the improved ADCC effect and CDC effect through a specific design of antibody structures, cf. EP 3 431 494 A1, EP 0 414 151 A1, US 5,082,774 A and US 5,169,762 A. However, in the development of other types of antibodies, the antibody-mediated ADCC effect and CDC effect are not always desired.

Nerve growth factor (NGF), the earliest found one among neurotrophic factors, is produced in the positions on neocortex and hippocampus, and consists of two α subunits, one β subunit and two γ subunits, which can promote the growth, development, differentiation, and maturation of central and peripheral neurons, maintain the normal functions of the nervous system, and accelerate the repair to the nervous system after damage. At present, the recombinant NGF is mainly used in treating various neuropathy and nerve damage.

Chinese hamster ovarian cell (CHO cell) is one of the most widely used expression systems of mammalian cells, which has good post-translational processing ability, so that the expression product can maintain its natural structure and activity. Currently, CHO has been used to express NGF and secrete the recombinant NGF outside the cell, and therefore NGF can be produced and obtained in a large scale (for example, Xu L et al., Expression, purification, and characterization of recombinant mouse nerve growth factor in Chinese hamster ovary cells. Protein Expr Purif, 2014, 104: 41-49 ; Wang XY et al., Characteristic element of matrix attachment region mediates vector attachment and enhances nerve growth factor expression in Chinese hamster ovary cells. Genet Mol Res, 2015, 14(3): 9191-9199). However, studies on the stable expression of NGF on the surface of a recombinant CHO cell have not been reported.

**SUMMARY OF THE INVENTION**

In order to be able to accurately detect the ADCC effect and CDC effect of an anti-NGF antibody, the inventors have developed a recombinant CHO cell stably expressing NGF on its surface through research, which is used as a target cell capable of being effectively used to evaluate the ADCC effect and CDC effect of the anti-NGF antibody.

The present invention is characterised in the claims.

In one aspect, the present application relates to a recombinant CHO cell, wherein NGF is stably expressed on the surface of the cell.

In another aspect, the present application relates to a method of constructing the above-mentioned recombinant CHO cell, comprising:

(1) cloning a nucleic acid sequence of a concatenated NGF, hinge region and transmembrane region into an expression vector to obtain a recombinant expression vector comprising the nucleic acid sequence;
(2) introducing the recombinant expression vector into a CHO cell to obtain the recombinant CHO cell.

In another aspect, the present application relates to a system for detecting an ADCC effect of an antibody, comprising:

the above-mentioned recombinant CHO cell, and
an effector cell.

In another aspect, the present application relates to a system for detecting a CDC effect of an antibody, comprising:

the above-mentioned recombinant CHO cell, and
a complement.

In yet another aspect, the present application relates to use of the above-mentioned recombinant CHO cell in detecting an ADCC effect or a CDC effect of an antibody. In yet another aspect, the present application relates to a method of detecting an ADCC effect of an antibody, comprising co-incubating the above-mentioned recombinant CHO cell as a target cell with an antibody to be detected and an effector cell.

In yet another aspect, the present application relates to a method of detecting a CDC effect of an antibody, comprising co-incubating the above-mentioned recombinant CHO cell as a target cell with an antibody to be detected and a complement.

In conventional recombinant NGF expression studies, a mammalian cell such as a CHO cell is usually used as an expression system to express and secrete NGF out of the cell, so that it is present in the supernatant in a free form similar to its natural form. In addition, some researchers have also tried to use prokaryotic systems (e.g., *E. coli*) to express NGF, and the expression product obtained is present as an insoluble inclusion body (Jiang Jing et al., Comparison of Recombinant Human $\beta$-NGF from E.coli and CHO in Biochemical Characters, China Biotechnology, 2006, 26(2): 8-12). There is considerable uncertainty on whether NGF, as a free protein rather than a transmembrane protein, can be expressed on the cell surface. Therefore, regarding the stable expression of NGF on the surface of the CHO cell, there have been no relevant reports in the art so far. In the present application, by concatenating the nucleic acid sequence of NGF with the nucleic acid sequence of an appropriate hinge region and transmembrane region and then introducing the same into a CHO cell, stable expression of NGF on the surface of the recombinant CHO cell is achieved, thereby making NGF present on the cell surface rather than being secreted into the culture fluid, and enabling the proper post-translational processing (including correct folding, glycosylation, etc.) of the expressed NGF and thus maintaining natural conformation and activity thereof. The recombinant CHO cell thus obtained has NGF on the cell surface and can be effectively used as a target cell for detecting the ADCC effect and CDC effect of an anti-NGF antibody, and thus can be used to detect the ADCC effect and CDC effect of the anti-NGF antibody.

**BRIEF DESCRIPTION OF THE DRAWINGS**

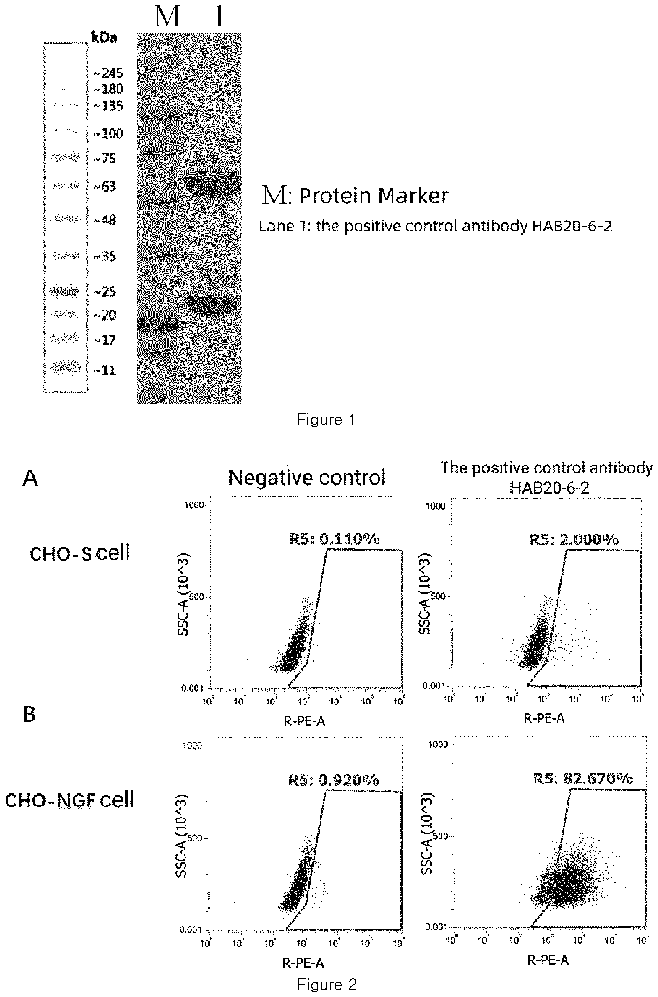

Figure 1 shows the results of SDS-PAGE detection of the positive control antibody HAB20-6-2. According to the results in Figure 1, it can be seen that the purity of the positive control antibody HAB20-6-2 prepared in Example 3 is greater than 90%.

Figure 2 shows the results of flow cytometry identification of CHO-NGF recombinant cell, wherein A in Figure 2 shows the flow cytometry detection results obtained by incubating the CHO-S cell as a mock control with anti-NGF positive control antibody HAB20-6-2, and the positive rate is 2%; B in Figure 2 shows the flow cytometry detection results obtained by incubating CHO-NGF cell constructed in Example 2 with anti-NGF positive control antibody HAB20-6-2, and the positive rate is 82.67%.

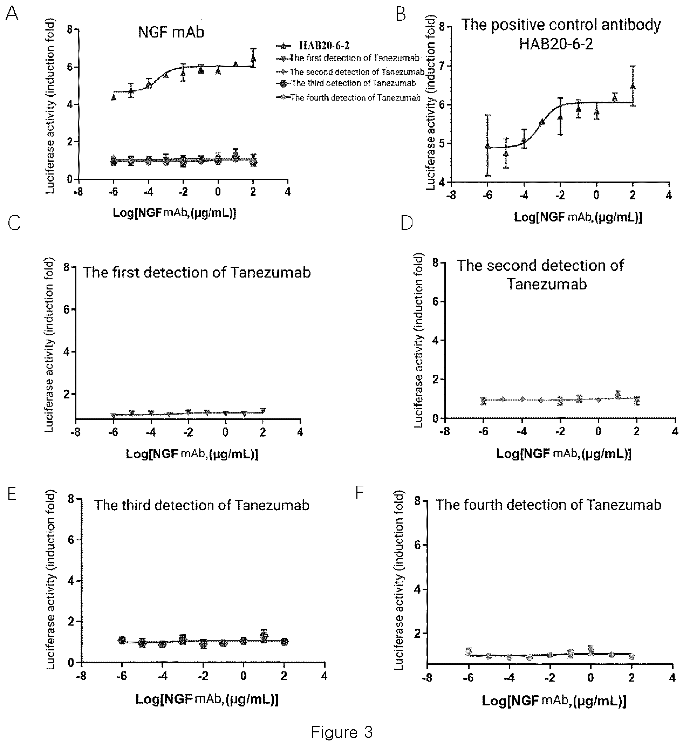

Figure 3 shows the results of the ADCC effect detection, wherein A: an overlay graph of the ADCC effect detection results of Tanezumab (4 detections) and the positive control antibody HAB20-6-2; B: the detection result of the positive control antibody HAB20-6-2; C: the first detection result of Tanezumab; D: the second detection result of Tanezumab; E: the third detection result of Tanezumab; F: the fourth detection result of Tanezumab.

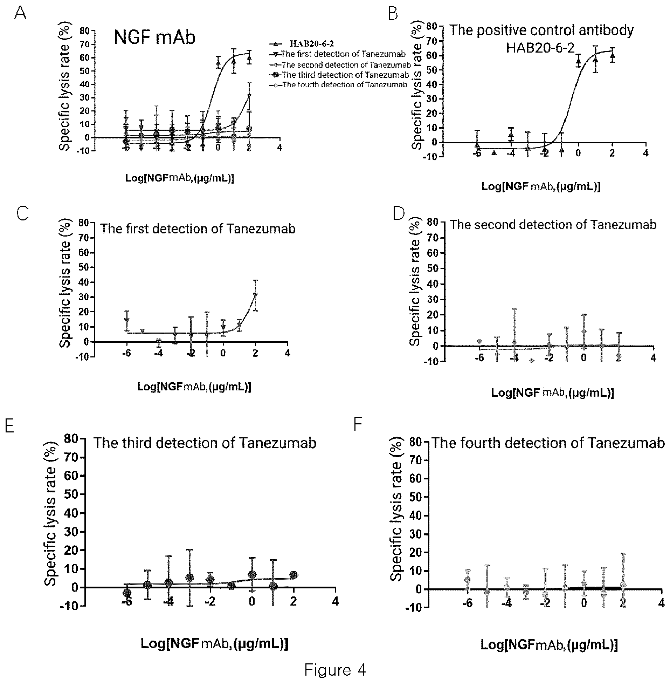

Figure 4 shows the results of the CDC effect detection, wherein A: an overlay graph of the CDC effect detection results of Tanezumab (4 detections) and the positive control antibody HAB20-6-2; B: the detection result of the positive control antibody HAB20-6-2; C: the first detection result of Tanezumab; D: the second detection result of Tanezumab; E: the third detection result of Tanezumab; F: the fourth detection result of Tanezumab.

**EMBODIMENTS OF THE INVENTION**

Hereinafter, the present application is further described in combination with particular embodiments, and the advantages and features of the present application will become clearer with description. However, these embodiments are merely exemplary and do not constitute any limitation to the scope of the present application.

Unless defined otherwise, the technical and scientific terms used herein have the same meanings as commonly understood by the person of ordinary skill in the art to which this disclosure belongs, which can be seen in, for example, Singleton et al., Dictionary of Microbiology and Molecular Biology 2nd ed., J. Wiley & Sons (New York, NY 1994); Sambrook et al., Molecular Cloning, A Laboratory Manual, Cold Springs Harbor Press (Cold Springs Harbor, NY 1989); Davis et al., Basic Methods in Molecular Biology, Elsevier Science Publishing Inc., New York, USA (2012); Abbas et al., Cellular and Molecular Immunology, Elsevier Science Health Science div (2009); He Wei et al., Medical Immunology (2nd Edition), People's Medical Publishing House Co., Ltd., 2010.

Unless stated otherwise, the terms "comprise", "comprises" and "comprising" or equivalents thereof ("contain", "contains", "containing", "include", "includes" and "including") herein are open-ended mode expressions which mean that other unspecified elements, components and steps may be included in addition to those already listed.

Unless stated otherwise, all the numbers used herein to express amounts of ingredients, measured values, or reaction conditions should be understood as being modified in all cases by the term "about". When being attached to a percentage, the term "about" can represent, e.g., $\pm 1\%$, preferably $\pm 0.5\%$, and more preferably $\pm 0.1\%$.

Unless explicitly indicated otherwise in the context, a singular term encompasses the plural indicating objects, and *vice versa*. Similarly, unless explicitly indicated otherwise in the context, the word "or" intends to include "and".

The percentage of identity (degree of homology) between sequences herein can be determined by aligning two sequences using, for example, freely accessible computer programs in the World Wide Web (e.g., www.ncbi.nlm.nih.gov) that are commonly used for this purpose (e.g., BLASTp or BLASTn with default settings).

Unless stated otherwise, the term "codon optimization" herein refers to a process of modifying nucleic acid sequences by replacing at least one codon of a natural sequence with a codon that is used more or most frequently in the host cell to improve expression in the host cell while maintaining the natural amino acid sequence. Various species have specific preference for specific codons of specific amino acids, and this codon preference (difference in codon use among different organisms) is typically related to the translation efficiency of mRNA.

In the present invention, "expression vector" refers to a vector comprising a desired coding sequence and an appropriate nucleic acid sequence essential to express the coding sequence operably linked in a particular host organism. The expression vector may preferably comprise one or more marker genes. In order to express the target sequence of the present invention, for example, any one of the following several expression control sequences can be used in the vector: replication origin, polyadenylation signal, promoter, enhancer, terminator, etc.

Since the first monoclonal antibody drug OKT3 (muromonab-CD3) was approved by the U.S. FDA in 1986, antibody drugs have been increasingly studied as an option for disease treatment. So far, FDA has approved hundreds of antibody drugs (https://www.nature.com/articles/d41573-021-00079-7). The mechanism by which antibodies activate the human immune system is very complex, mainly including antibody-dependent cell-mediated cytotoxicity (ADCC) and complement dependent cytotoxicity (CDC).

In general, since NGF *per se* is a free protein, the recombinant cell obtained by the construction capable of expressing NGF will secrete the expressed NGF out of the cell, so that it is present in the culture fluid in a free form substantially equivalent to the natural form, thereby making the NGF-expressing recombinant cell obtained by the common construction unsuitable as a target cell used in ADCC and CDC assays. There is considerable uncertainty on expressing NGF on the cell surface, and even if NGF is expressed on the surface of a recombinant cell, it is also unknown whether the expressed NGF can maintain the same natural conformation and activity as those of the free NGF. Therefore, recombinant CHO cells or other recombinant cells that can stably express NGF on their surfaces have not been reported.

In some embodiments, the present application relates to a recombinant CHO cell, wherein NGF is stably expressed on the surface of the cell.

Any NGF of known sequences can be used herein, such as the sequence available in NCBI, ENSMBL, or UniProt database, or a sequence obtained by performing codon optimization to a known sequence according to the codon preference of the host cell (existing software such as codon W can be used) can be employed. In some preferred embodiments, the NGF can be, but is not limited to, a mammalian NGF, such as a human NGF, a mouse NGF, a rat NGF, a bovine NGF, a horse NGF, a pig NGF, etc. The exemplary amino acid sequences and nucleic acid sequences of NGFs can be found in the following records: Katherine A. Fitzgerald et al., The Cytokine FactsBook and Webfacts (Second Edition), Academic Press, 2001; Wei Ma et al., Cloning and Sequencing of Matured Fragment of Human Never Growth Factor Gene, ACAD J XJ TU, 15(1), 2003: 62-65; YA Barde et al., The nerve growth factor family, Progress in Growth Factor Research, 2, 1990: 237-248; Wiesmann, C. et al., Nerve growth factor: structure and function. CMLS, Cell. Mol. Life Sci., 58, 2001: 748-759; Eric Adriaenssens et al., Nerve Growth Factor Is a Potential Therapeutic Target in Breast Cancer, Cancer Res., 68(2), 2008: January 15, 2008: 346-351; WO2021120900A1, WO2021093134A1, WO2021091363A1, WO2019201133A1, US2021079053A1, WO2010128398A1, US2014170137A1, etc.

In some preferred embodiments, the amino acid sequence of NGF comprises an amino acid sequence set forth in any one of SEQ ID NO: 1-SEQ ID NO: 3 or an amino acid sequence having at least 80% (e.g., 85%, 90%, 91%, 92%, 93%, 94%, 95%, 96%, 97%, 98% or 99% or higher) sequence identity thereto.

SEQ ID NO: 1, amino acid sequence of NGF

MSMLFYTLITAFLIGIQAEPHSESNVPAGHTIPQAHWTKLQHSLDTALRRARSAPAAAIAARVAGQTRNIT
VDPRLFKKRRLRSPRVLFSTQPPREAADTQDLDFEVGGAAPFNRTHRSKRSSSHPIFHRGEFSVCDSVSVW
VGDKTTATDIKGKEVMVLGEVNINNSVFKQYFFETKCRDPNPVDSGCRGIDSKHWNSYCTTTHTFVKAL
TMDGKQAAWRFIRIDTACVCVLSRKAVRRA

SEQ ID NO: 2, amino acid sequence 1 of mature NGF (mature NGF 1)

SSSHPIFHRGEFSVCDSVSVWVGDKTTATDIKGKEVMVLGEVNINNSVFKQYFFETKCRDPNPVDSGCRG
IDSKHWNSYCTTTHTFVKALTMDGKQAAWRFIRIDTACVCVLSRKAVRRA

SEQ ID NO: 3, amino acid sequence 2 of mature NGF (mature NGF 2)

SSSHPIFHRGEFSVCDSVSVWVGDKTTATDIKGKEVMVLGEVNINNSVFKQYFFETKCRDPNPVDSGCRG
IDSKHWNSYCTTTHTFVKALTMDGKQAAWRFIRIDTACVCVLSRKAVR

In some preferred embodiments, the genome of the cell is integrated with a nucleic acid sequence of NGF.

In some preferred embodiments, the nucleic acid sequence of NGF comprises a nucleic acid sequence set forth in any one of SEQ ID NO: 4-SEQ ID NO: 6 or a nucleic acid sequence having at least 80% (e.g., 85%, 90%, 91%, 92%, 93%, 94%, 95%, 96%, 97%, 98% or 99% or higher) sequence identity thereto.

SEQ ID NO: 4, nucleic acid sequence of NGF

ATGAGCATGCTGTTCTATACCCTGATCACCGCCTTTCTGATCGGCATCCAGGCCGAGCCTCACAGCGA
GTCTAATGTGCCTGCCGGCCACACAATCCCTCAGGCTCACTGGACAAAGCTGCAGCACAGCCTGGAT
ACAGCTCTGCGGAGAGCCAGATCTGCTCCTGCCGCTGCTATTGCCGCTAGAGTGGCTGGCCAGACCA
GAAACATCACCGTGGATCCCCGGCTGTTCAAGAAGCGGAGACTGAGAAGCCCCAGAGTGCTGTTCA
GCACCCAGCCTCCAAGAGAGGCCGCCGATACACAGGACCTGGATTTTGAAGTTGGCGGCGCTGCCCC
TTTCAACAGAACCCACAGAAGCAAGCGGAGCAGCTCTCACCCCATCTTCCACAGAGGCGAGTTCAG
CGTGTGCGACTCCGTGTCTGTGTGGGTCGGAGATAAGACCACCGCCACCGACATCAAGGGCAAAGAA
GTGATGGTCCTGGGCGAAGTGAACATCAACAACAGCGTGTTCAAGCAGTACTTCTTCGAGACAAAGT

GCAGGGACCCCAATCCTGTGGACAGCGGCTGTAGAGGCATCGACAGCAAGCACTGGAACTCCTACTG
CACCACCACACACACCTTCGTGAAGGCCCTGACCATGGATGGAAAACAGGCCGCCTGGCGGTTCATC
AGAATCGATACCGCTTGCGTGTGCGTGCTGAGCAGAAAAGCCGTTAGAAGGGCC

SEQ ID NO: 5, nucleic acid sequence of mature NGF 1

AGCAGCTCTCACCCCATCTTCCACAGAGGCGAGTTCAGCGTGTGCGACTCCGTGTCTGTGTGGGTCG
GAGATAAGACCACCGCCACCGACATCAAGGGCAAAGAAGTGATGGTCCTGGGCGAAGTGAACATCA
ACAACAGCGTGTTCAAGCAGTACTTCTTCGAGACAAAGTGCAGGGACCCCAATCCTGTGGACAGCGG
CTGTAGAGGCATCGACAGCAAGCACTGGAACTCCTACTGCACCACCACACACACCTTCGTGAAGGCC
CTGACCATGGATGGAAAACAGGCCGCCTGGCGGTTCATCAGAATCGATACCGCTTGCGTGTGCGTGCT
GAGCAGAAAAGCCGTTAGAAGGGCC

SEQ ID NO: 6, nucleic acid sequence of mature NGF 2

AGCAGCTCTCACCCCATCTTCCACAGAGGCGAGTTCAGCGTGTGCGACTCCGTGTCTGTGTGGGTCG
GAGATAAGACCACCGCCACCGACATCAAGGGCAAAGAAGTGATGGTCCTGGGCGAAGTGAACATCA
ACAACAGCGTGTTCAAGCAGTACTTCTTCGAGACAAAGTGCAGGGACCCCAATCCTGTGGACAGCGG
CTGTAGAGGCATCGACAGCAAGCACTGGAACTCCTACTGCACCACCACACACACCTTCGTGAAGGCC
CTGACCATGGATGGAAAACAGGCCGCCTGGCGGTTCATCAGAATCGATACCGCTTGCGTGTGCGTGCT
GAGCAGAAAAGCCGTTAGA

In some preferred embodiments, the genome of the cell is integrated with a nucleic acid sequence of a concatenated NGF, hinge region and transmembrane region (also known as "transmembrane domain").

In further preferred embodiments, the amino acid sequence of the hinge region has 3 or more amino acids, 10 or

**EP 4 230 732 B1**

more amino acids, 20 or more amino acids, 30 or more amino acids, 40 or more amino acids, 50 or more amino acids, or 100 or less amino acids, 90 or less amino acids, 80 or less amino acids, 70 or less amino acids, 60 or less amino acids, or amino acids within the range of the above any two numerical values, such as 3-100 amino acids, 10-90 amino acids, 10-80 amino acids, 10-70 amino acids, 10-60 amino acids, 10-50 amino acids, and 12-45 amino acids.

In further preferred embodiments, the amino acid sequence of the transmembrane region has 10 or more amino acids, 20 or more amino acids, 30 or more amino acids, or 60 or less amino acids, 50 or less amino acids, 40 or less amino acids, or amino acids within the range of the above any two numerical values, such as 10-60 amino acids, 10-50 amino acids, 10-40 amino acids, 10-30 amino acids, 15-30 amino acids, 20-30 amino acids, and 20-25 amino acids.

In further preferred embodiments, the hinge region may be a CD8 hinge region (e.g., CD8α hinge region) or a human IgG1 hinge region.

In further preferred embodiments, the transmembrane region may be a CD8 transmembrane region, a PGFRA transmembrane region, or a CD80 transmembrane region.

In some preferred embodiments, the amino acid sequence of the CD8 hinge region comprises an amino acid sequence set forth in SEQ ID NO: 7 or an amino acid sequence having at least 80% (e.g., 85%, 90%, 91%, 92%, 93%, 94%, 95%, 96%, 97%, 98% or 99% or higher) sequence identity thereto.

SEQ ID NO: 7, amino acid sequence of the CD8 hinge region
TTTPAPRPPTPAPTIASQPLSLRPEACRPAAGGAVHTRGLDFACD

In some preferred embodiments, the amino acid sequence of the human IgG1 hinge region comprises an amino acid sequence set forth in SEQ ID NO: 8 or an amino acid sequence having at least 80% (e.g., 85%, 90%, 91%, 92%, 93%, 94%, 95%, 96%, 97%, 98% or 99% or higher) sequence identity thereto.

SEQ ID NO: 8, amino acid sequence of the human IgG1 hinge region
EPKSCDKTHTCP

In some preferred embodiments, the nucleic acid sequence encoding the CD8 hinge region comprises a nucleic acid sequence set forth in SEQ ID NO: 9 or a nucleic acid sequence having at least 80% (e.g., 85%, 90%, 91%, 92%, 93%, 94%, 95%, 96%, 97%, 98% or 99% or higher) sequence identity thereto.

SEQ ID NO: 9, nucleic acid sequence of the CD8 hinge region

ACCACTACCCCAGCACCGAGGCCACCCACCCCGGCTCCTACCATCGCCTCCCAGCCTCTGTCCCTGCG
TCCGGAGGCATGTAGACCCGCAGCTGGTGGGGCCGTGCATACCCGtGGTCTTGACTTCGCCTGCGAT

In some preferred embodiments, the amino acid sequence of the CD8 transmembrane region comprises an amino acid sequence set forth in SEQ ID NO: 10 or an amino acid sequence having at least 80% (e.g., 85%, 90%, 91%, 92%, 93%, 94%, 95%, 96%, 97%, 98% or 99% or higher) sequence identity thereto.

SEQ ID NO: 10, amino acid sequence of the CD8 transmembrane region
IYIWAPLAGTCGVLLLSLVITLYC

In some preferred embodiments, the amino acid sequence of the PGFRA transmembrane region comprises an amino acid sequence set forth in SEQ ID NO: 11 or an amino acid sequence having at least 80% (e.g., 85%, 90%, 91%, 92%, 93%, 94%, 95%, 96%, 97%, 98% or 99% or higher) sequence identity thereto.

SEQ ID NO: 11, amino acid sequence of the PGFRA transmembrane region
AAVLVLLVIVIISLIVLVVIW

In some preferred embodiments, the amino acid sequence of the CD80 transmembrane region comprises an amino acid sequence set forth in SEQ ID NO: 12 or an amino acid sequence having at least 80% (e.g., 85%, 90%, 91%, 92%, 93%, 94%, 95%, 96%, 97%, 98% or 99% or higher) sequence identity thereto.

SEQ ID NO: 12, amino acid sequence of the CD80 transmembrane region
LLPSWAITLISVNGIFVICCL

In some preferred embodiments, the nucleic acid sequence encoding the CD8 transmembrane region comprises a nucleic acid sequence set forth in SEQ ID NO: 13 or a nucleic acid sequence having at least 80% (e.g., 85%, 90%, 91%, 92%, 93%, 94%, 95%, 96%, 97%, 98% or 99% or higher) sequence identity thereto.

SEQ ID NO: 13, nucleic acid sequence of the CD8 transmembrane region

ATCTACATTTGGGCCCCTCTGGCTGGTACTTGCGGGGTCCTGCTGCTTTCACTCGTGATCACTCTTTAC
TGT

In some preferred embodiments, the hinge region is a CD8 hinge region, and the transmembrane region is a CD8 transmembrane region, a PGFRA transmembrane region, or a CD80 transmembrane region. For example, in some instances, the hinge region is a CD8 hinge region, and the transmembrane region is a CD8 transmembrane region. Or for example, the hinge region is a CD8 hinge region, and the transmembrane region is a PGFRA transmembrane region. Or for example, the hinge region is a CD8 hinge region, and the transmembrane region is a CD80 transmembrane region.

In some preferred embodiments, the hinge region is a human IgG1 hinge region, and the transmembrane region is a CD8 transmembrane region, a PGFRA transmembrane region, or a CD80 transmembrane region. For example, in some instances, the hinge region is a human IgG1 hinge region, and the transmembrane region is a CD8 transmembrane region. Or for example, the hinge region is a human IgG1 hinge region, and the transmembrane region is a PGFRA transmembrane region. Or for example, the hinge region is a human IgG1 hinge region, and the transmembrane region is a CD80 transmembrane region.

In some preferred embodiments, the nucleic acid sequence of the concatenated NGF, hinge region and transmembrane region is a nucleic acid sequence of concatenated NGF, CD8 hinge region and CD8 transmembrane region.

In some embodiments, the present application relates to a method of constructing the above-mentioned recombinant CHO cell, comprising:

(1) cloning a nucleic acid sequence of a concatenated NGF, hinge region and transmembrane region into an expression vector to obtain a recombinant expression vector comprising the nucleic acid sequence;
(2) introducing the recombinant expression vector into a CHO cell to obtain the recombinant CHO cell.

In some preferred embodiments, the NGF can be, but is not limited to, a mammalian NGF, such as a human NGF, a mouse NGF, a rat NGF, a bovine NGF, a horse NGF, a pig NGF, etc.

In some preferred embodiments, the amino acid sequence of NGF comprises an amino acid sequence set forth in any one of SEQ ID NO: 1-SEQ ID NO: 3 or an amino acid sequence having at least 80% (e.g., 85%, 90%, 91%, 92%, 93%, 94%, 95%, 96%, 97%, 98% or 99% or higher) sequence identity thereto.

In some preferred embodiments, the nucleic acid sequence of NGF comprises a nucleic acid sequence set forth in any one of SEQ ID NO: 4-SEQ ID NO: 6 or a nucleic acid sequence having at least 80% (e.g., 85%, 90%, 91%, 92%, 93%, 94%, 95%, 96%, 97%, 98% or 99% or higher) sequence identity thereto.

In further preferred embodiments, the amino acid sequence of the hinge region has 3 or more amino acids, 10 or more amino acids, 20 or more amino acids, 30 or more amino acids, 40 or more amino acids, 50 or more amino acids, or 100 or less amino acids, 90 or less amino acids, 80 or less amino acids, 70 or less amino acids, 60 or less amino acids, or amino acids within the range of the above any two numerical values, such as 3-100 amino acids, 10-100 amino acids, 0-90 amino acids, 10-80 amino acids, 10-70 amino acids, 10-60 amino acids, 10-50 amino acids, and 12-45 amino acids.

In a further preferred embodiment, the amino acid sequence of the transmembrane region has 10 or more amino acids in length, 20 or more amino acids, 30 or more amino acids, or 60 or less amino acids, 50 or less amino acids, 40 or less amino acids, or amino acids within the range of the above any two numerical values, such as 10-60 amino acids, 10-50 amino acids, 10-40 amino acids, 10-30 amino acids, 15-30 amino acids, 20-30 amino acids, and 20-25 amino acids.

In some preferred embodiments, the hinge region may be a CD8 hinge region (e.g., CD8α hinge region) or a human IgG1 hinge region.

In some preferred embodiments, the transmembrane region may be a CD8 transmembrane region, a PGFRA transmembrane region, or a CD80 transmembrane region.

In some preferred embodiments, the amino acid sequence of the CD8 hinge region comprises an amino acid sequence set forth in SEQ ID NO: 7 or an amino acid sequence having at least 80% (e.g., 85%, 90%, 91%, 92%, 93%, 94%, 95%, 96%, 97%, 98% or 99% or higher) sequence identity thereto. In some preferred embodiments, the amino acid sequence of the human IgG1 hinge region comprises an amino acid sequence set forth in SEQ ID NO: 8 or an amino acid sequence having at least 80% (e.g., 85%, 90%, 91%, 92%, 93%, 94%, 95%, 96%, 97%, 98% or 99% or higher) sequence identity thereto.

In some preferred embodiments, the nucleic acid sequence encoding the CD8 hinge region comprises a nucleic acid sequence set forth in SEQ ID NO: 9 or a nucleic acid sequence having at least 80% (e.g., 85%, 90%, 91%, 92%, 93%, 94%, 95%, 96%, 97%, 98% or 99% or higher) sequence identity thereto.

In some preferred embodiments, the amino acid sequence of the CD8 transmembrane region comprises an amino acid sequence set forth in SEQ ID NO: 10 or an amino acid sequence having at least 80% (e.g., 85%, 90%, 91%, 92%, 93%, 94%, 95%, 96%, 97%, 98% or 99% or higher) sequence identity thereto. In some preferred embodiments, the amino acid sequence of the PGFRA transmembrane region comprises an amino acid sequence set forth in SEQ ID NO: 11 or an amino acid sequence having at least 80% (e.g., 85%, 90%, 91%, 92%, 93%, 94%, 95%, 96%, 97%, 98% or 99% or higher) sequence identity thereto. In some preferred embodiments, the amino acid sequence of the CD80 transmembrane region comprises an amino acid sequence set forth in SEQ ID NO: 12 or an amino acid sequence having at least 80% (e.g., 85%, 90%, 91%, 92%, 93%, 94%, 95%, 96%, 97%, 98% or 99% or higher) sequence identity thereto.

In some preferred embodiments, the nucleic acid sequence encoding the CD8 transmembrane region comprises a nucleic acid sequence set forth in SEQ ID NO: 13 or a nucleic acid sequence having at least 80% (e.g., 85%, 90%, 91%, 92%, 93%, 94%, 95%, 96%, 97%, 98% or 99% or higher) sequence identity thereto.

In some preferred embodiments, the hinge region is a CD8 hinge region, and the transmembrane region is a CD8 transmembrane region, a PGFRA transmembrane region, or a CD80 transmembrane region. For example, in some instances, the hinge region is a CD8 hinge region, and the transmembrane region is a CD8 transmembrane region. Or for example, the hinge region is a CD8 hinge region, and the transmembrane region is a PGFRA transmembrane region. Or for example, the hinge region is a CD8 hinge region, and the transmembrane region is a CD80 transmembrane region.

In some preferred embodiments, the hinge region is a human IgG1 hinge region, and the transmembrane region is a CD8 transmembrane region, a PGFRA transmembrane region, or a CD80 transmembrane region. For example, in some instances, the hinge region is a human IgG1 hinge region, and the transmembrane region is a CD8 transmembrane region. Or for example, the hinge region is a human IgG1 hinge region, and the transmembrane region is a PGFRA transmembrane region. Or for example, the hinge region is a human IgG1 hinge region, and the transmembrane region is a CD80 transmembrane region.

In some preferred embodiments, the nucleic acid sequence of the concatenated NGF, hinge region and transmembrane region is a nucleic acid sequence of a concatenated NGF, CD8 hinge region and CD8 transmembrane region.

In some preferred embodiments, the expression vector can be, but is not limited to, a lentiviral expression vector, an adenovirus vector, an adeno-associated virus vector, a retroviral vector, a poxvirus vector or a herpes simplex virus vector. As an example of the lentiviral expression vector, the lentiviral expression vector can be, for example, a Lenti-CMV-puro lentiviral expression vector, a lentiviral expression vector of pCDH series, or a lentiviral expression vector of pLenti series, but not limited thereto.

In some preferred embodiments, in step (2), the recombinant expression vector and a virus packaging plasmid are co-transfected into a packaging cell. After culturing, a virus comprising the nucleic acid sequence is prepared, and then the virus is used to transfect a CHO cell to obtain the recombinant CHO cell.

In further preferred embodiments, the virus packaging plasmid can be pMD2.G, psPAX2, Lenti-packaging Mix, pCMV-dR8.91, pCMV-dR8.74, pLP1, pLP2, pVSV-G, etc., but not limited thereto.

In further preferred embodiments, the packaging cell may be a 293T cell, a 293FT cell, etc., but not limited thereto.

Unless stated otherwise, cell culture herein is conducted using common culture conditions and common cell culture media (such as 1640 culture medium, DMEM culture medium, MEM culture medium, F12 culture medium, etc.) in the art, which can be found in, for example, Lan Rong et al., Cell Culture Technology, Chemical Industry Press Co., Ltd., 2007; R.I. Fershney, Culture of Animal Cells: A Manual of Basic Technique and Specialized Applications (Sixth Edition), John Wiley & Sons, Inc; 2010; Liu Bin et al., Cell Culture (Third Edition), World Publishing Corporation, 2018; the contents of each of which are incorporated herein by reference in their entireties.

Transfection is one of the commonly used means known in the art for introducing a target exogenous DNA (extracted from a donor of interest or directly synthesized) into host cells. Virus-mediated transfection is one of the commonly used transfection methods in the art, which uses a method of infecting cells with a virus packaging a desired exogenous gene enabling the exogenous gene of interest entering the cell. The method has the advantages such as the highest transfection efficiency and low cytotoxicity.

In some embodiments, the present application relates to a system for detecting an ADCC effect of an antibody, comprising:

> the above-mentioned recombinant CHO cell, and
> an effector cell.

In the present disclosure, the effector cell can be any common effector cell known in the art. As an example, in some preferred embodiments, the effector cell can include, but are not limited to, effector T cell, NK cell, phagocyte, eosinophil, basophil and mast cell, such as Jurkat-NFAT-Luciferase-CD16 cell, primary NK cell, etc.

In some preferred embodiments, the effector-target ratio (E:T) of the effector cell to the recombinant CHO cell by number can be 1:1 to 1:20, e.g., 1:5.

In some preferred embodiments, the system further includes a luciferase substrate. As a typical luciferase, firefly luciferase is a protein with a molecular weight of about 61kDa, which can catalyze the oxidation of luciferin to oxyluciferin in the presence of ATP, magnesium ions and oxygen, and emits biofluorescence during the oxidation process.

In some embodiments, the present application relates to a system for detecting a CDC effect of an antibody, comprising:

> the above-mentioned recombinant CHO cell, and
> a complement.

Complement, also known as complement system, is a protein present in human and animal serum or tissue fluid that can mediate immune response and inflammation, including more than 30 soluble proteins and membrane binding proteins. In some preferred embodiments, the complement is a complement serum comprising complement proteins C1-C9.

In some preferred embodiments, the system further comprises a luciferase substrate. In some embodiments, the present application relates to use of the above-mentioned recombinant CHO cell in detecting an ADCC effect or a CDC effect of an antibody.

In some embodiments, the present application relates to a method of detecting an ADCC effect of an antibody, comprising co-incubating the above-mentioned recombinant CHO cell as a target cell with an antibody to be detected and an effector cell. In further preferred embodiments, the effector-target ratio of the effector cell to the target cell by number can be 1:1 to 1:20, e.g., 1:5. In some preferred embodiments, the method further comprises adding a luciferase substrate to the system after co-incubation.

In some embodiments, the present application relates to a method of detecting a CDC effect of an antibody, comprising co-incubating the above-mentioned recombinant CHO cell as a target cell with an antibody to be detected and a complement. In further preferred embodiments, the complement is a complement serum comprising complement proteins C1-C9. In some preferred embodiments, the method further comprises adding a luciferase substrate to the system after co-incubation.

## EXAMPLES

It should be illustrated that the operational steps involved in the present application, such as the design, synthesis, and cloning of genes, the linkage of gene fragments, the construction of expression vectors, analysis and identification of sequences, the construction of a recombinant cell, and the isolation and purification of expression products, can be performed in accordance with techniques known in the art (e.g., see the records in CURRENT PROTOCOLS IN MOLECULAR BIOLOGY). Unless specified otherwise, the technical means used in the examples are common means well known by the skilled in the art. Unless stated otherwise, the reagents, materials, and devices used in the following examples are commercially available.

The main reagents, consumable materials and devices used in the following examples and sources thereof are shown in the table below. The skilled in the art can use any other reagents, consumable materials and devices with equivalent effects.

| Reagents or consumable materials | Manufacturers | Cat No. | Lot No. |
|---|---|---|---|
| PBS | Shanghai BasalMedia Technologies Co., Ltd | B210KJ | M210807 |
| Transfection reagent LVTransm | iCarTAB | LVTran 100 | 2020041701 |
| FBS | BI | 04-001-1 ACS | 2033119 |
| DMEM medium with high glucose | Gbico | 10566024 | 2192449 |
| Lenti-CMV-puro lentiviral expression vector | iCarTab Biotechnology (Suzhou) Co., Ltd. | / | / |
| CHOGrow CD1 serum-free medium | Basalmedia | H110KJ | J430808 |
| Bright-Glo™ Luciferase Assay System | Promega | E2610 | 08968 |
| Cell Counting-Lite 3D Luminescent Cell Viability Assay | Vazyme | DD1102-01 | 7E401B0 |
| Human complement serum | Sigma | S1764-1mL | SLBL5471V |
| 50mL Falcon centrifugal tube | Promethe | PCT010500 | 220210102 |
| 15m L Falcon centrifugal tube | Promethe | PCT010150 | 20201313 |
| 6 well plate | Thermo | 140675 | 60337385 |
| 96 well plate | Thermo | 136101 | 162032 |

| Devices | Manufacturers | Models |
|---|---|---|
| Ordinary optical inverted microscope | Motic | AE-200 |
| Desktop centrifuge | Thermo | ST41 |
| Biosafety cabinet | Haier | HR40-IIA2 |

(continued)

| Devices | Manufacturers | Models |
|---|---|---|
| Flow cytometer | Thermo | Attune Nxt flow cytometer |
| $CO_2$ incubator | Thermo | 3111 |
| Multifunctional microplate reader | Tecan | M1000 PRO |

**Example 1 Construction of a NGF overexpression lentiviral vector and preparation of lentivirus**

The nucleic acid sequence of NGF could be concatenated with the nucleic acid sequences encoding the hinge region and the transmembrane region and inserted into an appropriate expression vector, thereby achieving the heterologous expression of the above nucleic acid sequences. Regarding NGF, a NGF from mammals could be selected, such as a human NGF, a mouse NGF, a rat NGF, a bovine NGF, a horse NGF, a pig NGF, etc., and a NGF and nucleic acid sequence thereof could be synthesized based on the known sequences disclosed by commonly used databases of nucleic acid sequences and protein sequences, such as NCBI, ENSMBL or UniProt.

Here, a human NGF was taken as an example, and a CD8 hinge region and a CD8 transmembrane region were used to be concatenated with the NGF. Specifically, the full-length sequence of the human NGF was codon optimized according to the codon preference of the host cell CHO-S cell to obtain NGF with an amino acid sequence set forth in SEQ NO ID: 1 (the corresponding nucleic acid sequence was set forth in SEQ ID NO: 4), which was concatenated with the CD8 hinge region (the amino acid sequence thereof was set forth in SEQ ID NO: 7; the nucleic acid sequence thereof was set forth in SEQ ID NO: 9) and the CD8 transmembrane region (the amino acid sequence thereof was set forth in SEQ ID NO: 10; the nucleic acid sequence thereof was set forth in SEQ ID NO: 13). Gene synthesis was conducted after concatenation and the synthesized gene fragments were subcloned to Lenti-CMV-puro lentiviral expression vector (iCarTab Biotechnology (Suzhou) co.ltd). The target gene overexpression vector Lenti-CMV-NGF-Full (CD8-TM) vector was prepared; and after being sequenced to verify that the sequence was correct, the endotoxin-removing plasmid was prepared for future use.

Next, lentivirus was prepared by the steps as follows:

1. A 15cm cell culture dish was prepared, 293T cells (Jennio Biological) were inoculated thereto at $5 \times 10^6$ cells/dish, a complete medium (DMEM medium with high glucose supplemented with 10% FBS) was added thereinto, and it was cultured in a 37°C, 5% $CO_2$ incubator overnight.

2. The transfection reagent LVTransm, Lenti-CMV-NGF-Full (CD8-TM) expression vector and lentiviral packaging plasmid Lenti-packaging Mix were taken out from the refrigerator. After being thawed at room temperature, they were pipette up and down by a pipettor to mix the same thoroughly. The transfection complex was prepared by the following process: $1 \times$ PBS or HBSS buffer was taken and warmed to room temperature; then 2 mL of PBS or HBSS buffer was taken and added into a well of a 6-well plate; 10 μg of Lenti-CMV-NGF-Full (CD8-TM) expression vector, 30 μL of Lenti-packaging Mix were added thereto, respectively; after it was pipette up and down by a pipettor to mix the same thoroughly, 50 μL of LVTransm was added thereto; it was pipette up and down using the pipettor immediately to mix well; and let it stand at room temperature for 10-15 minutes.

3. The above transfection complex was added dropwise into the 15 cm cell culture dish in step 1; then gently shook the culture dish to mix well; the culture dish was placed and cultured in a 37°C, 5% $CO_2$ incubator for 6 to 8 hours, followed by removing the medium comprising the transfection reagent and replacing it with the fresh complete medium, and the culture dish was put back into the incubator to continue the culture.

4. After continuous culture for 24 hours, the culture medium supernatant comprising virus in the culture dish was collected into a 50 mL centrifugal tube; about 25 mL of the fresh complete medium was re-added to the culture dish, and the culture was continued for 24 hours.

5. The culture medium supernatant comprising virus in the culture dish was collected into a 50 mL centrifugal tube; about 25 mL of the fresh complete medium was re-added to the culture dish, and the culture wad continued for 24 hours;

6. The culture medium supernatant comprising virus in the culture dish was collected and mixed with the two culture medium supernatants collected before;

7. After the above culture medium supernatant was filtered with a 0.45μm PES filter membrane, the filtrate was transferred to a sterile centrifugal tube and centrifuged at 50,000×g, 4°C for 2 hours. After centrifugation, the liquid in the centrifugal tube was carefully sucked off in the biosafety cabinet, 1 mL of PBS buffer was added to resuspend the precipitate, and the resulting Lenti-CMV-NGF-Full (CD8-TM) overexpression lentivirus was stored at -80°C.

**Example 2 Construction of the recombinant CHO cell**

Using the Lenti-CMV-NGF-Full (CD8-TM) overexpression lentivirus prepared in Example 1, recombinant CHO cells (also known as "CHO-NGF recombinant cells" or "CHO-NGF cells") that can stably express NGF on the cell surface were prepared by the following process:

1. CHO-S cells were resuscitated from liquid nitrogen and cultured in a 37°C, 5% $CO_2$ incubator using CHOGrow CD1 serum-free medium. The cells were passed 5 times consecutively, so that the cells were in a logarithmic growth stage.

2. A new 6-well plate was taken out, the above CHO-S cells in the logarithmic growth stage were inoculated into the 6-well plate at a density of $5×10^6$ cells/well, and 3mL of CHOGrow CD1 serum-free medium was added thereto. And then according to MOI=5, the required amount of virus was calculated based on the number of inoculated cells and the virus titer, and the calculation formula was as follows:

$$\text{Amount of virus (mL)} = (\text{number of cells} × \text{MOI})/\text{virus titer}$$

3. The Lenti-CMV-NGF-Full (CD8-TM) overexpression lentivirus prepared in Example 1 was added into the 6-well plate in the amount calculated by the above formula, and after being gently mixed with a pipettor, the 6-well plate was placed in a centrifuge and centrifuged at 800×g under room temperature for 1 hour.

4. After centrifugation, the 6-well plate was taken out and placed in a 37°C, 5% $CO_2$ incubator to continue the culture for 24 hours.

5. After 24h of culture, the culture medium in the 6-well plate was replaced with the fresh CHOGrow CD1 serum-free medium and cultured continuously for 24 hours.

6. The culture medium in the 6-well plate was replaced with the CHOGrow CD1 serum-free medium comprising 8μg/mL of Puromycin, and it was cultured continuously for 5 days until all the uninfected CHO-S cells were killed.

7. The remaining living cells were continued the expanded culture, and then the cells were collected to give the constructed recombinant CHO cells.

**Example 3 Flow cytometry identification of the recombinant CHO cell**

Using a positive control antibody, the recombinant CHO cells constructed in Example 2 were identified by the flow cytometry. Wherein, the positive control antibody HAB20-6-2 was prepared according to the following process:

1. Starting from Tanezumab, an anti-NGF antibody of IgG2 subtype developed by Pfizer, according to the sequence information of the antibody Tanezumab (the heavy chain sequence thereof is set forth in SEQ NO ID: 14; the light chain sequence thereof is set forth in SEQ NO ID: 15), the constant region sequence in the Fc segment of the antibody Tanezumab was replaced with the constant region sequence of human IgG1 subtype (the amino acid sequence is set forth in SEQ NO ID: 16; the nucleotide sequence is set forth in SEQ NO ID: 17), and the rest remained unchanged. A Human IgG1 subtype recombinant antibody expression vector was constructed by using pcDNA3.1-hIgG1 vector (prepared by iCarTab Biotechnology (Suzhou) Co., Ltd.), and stored in a refrigerator and frozen for future use.

2. The transfection reagent LVTransm and Human IgG1 subtype recombinant antibody expression vector were taken out from the refrigerator. After being thawed at room temperature, the both were pipette up and down by a pipettor to mix well, respectively. 1 × PBS or HBSS buffer was taken out and warmed to room temperature. Then, 2mL of PBS or HBSS buffer was taken and added into a well of a 6-well plate, into which 90μg of Human IgG1 subtype recombinant antibody expression vector (the ratio of the light chain to the heavy chain was 2:1) was added, and it was pipette up and down by a pipettor to mix well, followed by adding 270μL of LVTransm thereto, and it was pipette up and down using a pipettor immediately to mix well. Let it stand at room temperature for 10 minutes to give a complex.

3. The above obtained complex was added to 50 mL of 293F cells, which was shaken gently to mix well. The cells were placed in a 37°C, 5% $CO_2$ incubator and cultured at 130RPM for 6 to 8 hours, 50mL of fresh 293F medium was then added to the cells, and the cells were put back to the incubator to culture continuously.

4. After 7 days of continuous culture, the culture medium supernatant was collected by centrifugation and filtered with a 0.45μm filter membrane, then the filtrate was transferred to a sterile centrifugal tube, and the antibody was purified using a Protein A affinity column to obtain the positive control antibody HAB20-6-2.

5. Purity of the protein was detected by SDS-PAGE.

The detection results were shown in Figure 1. According to the SDS-PAGE detection results, the purity of the positive control antibody HAB20-6-2 prepared above was greater than 90% and could be used in an ADCC effect assay.

SEQ NO ID: 14: Heavy chain sequence of Tanezumab

QVQLQESGPGLVKPSETLSLTCTVSGFSLIGYDLNWIRQPPGKGLEWIGIIWGDGTTDYNSAVKSRVTISKD
TSKNQFSLKLSSVTAADTAVYYCARGGYWYATSYYFDYWGQGTLVTVSSASTKGPSVFPLAPCSRSTSES
TAALGCLVKDYFPEPVTVSWNSGALTSGVHTFPAVLQSSGLYSLSSVVTVPSSNFGTQTYTCNVDHKPSNT
KVDKTVERKCCVECPPCPAPPVAGPSVFLFPPKPKDTLMISRTPEVTCVVVDVSHEDPEVQFNWYVDGVE
VHNAKTKPREEQFNSTFRVVSVLTVVHQDWLNGKEYKCKVSNKGLPSSIEKTISKTKGQPREPQVYTLPP
SREEMTKNQVSLTCLVKGFYPSDIAVEWESNGQPENNYKTTPPMLDSDGSFFLYSKLTVDKSRWQQGNVF
SCSVMHEALHNHYTQKSLSLSPGK

SEQ NO ID: 15: Light chain sequence of Tanezumab

DIQMTQSPSSLSASVGDRVTITCRASQSISNNLNWYQQKPGKAPKLLIYYTSRFHSGVPSRFSGSGSGTDFT
FTISSLQPEDIATYYCQQEHTLPYTFGQGTKLEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

SEQ NO ID: 16: Amino acid sequence of human IgG1 constant region

ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGK

SEQ NO ID: 17: Nucleic acid sequence of human IgG1 constant region

GCCTCTACAAAGGGCCCCTCCGTTTTTCCACTGGCTCCCAGCAGCAAGTCTACCTCTGGTGGAACAG
CCGCTCTGGGCTGCCTGGTCAAGGATTACTTTCCCGAGCCAGTGACCGTGTCCTGGAAcTCTGGCGCT
CTGACATCTGGCGTGCACACATTTCCAGCCGTGCTGCAGTCTAGCGGCCTGTACTCTCTGAGCAGCGT
GGTCACAGTGCCTAGCTCTAGCCTGGGCACCCAGACCTACATCTGCAATGTGAACCACAAGCCTAGC
AACACCAAGGTGGACAAGAAGGTGGAACCCAAGAGCTGCGACAAGACCCACACCTGTCCTCCATGT
CCTGCTCCAGAACTGCTCGGCGGACCTTCCGTGTTTCTGTTCCCTCCAAAGCCTAAGGACACCCTGAT
GATCAGCAGAACCCCTGAAGTGACCTGCGTGGTGGTGGATGTGTCCCACGAGGAcCCCGAAGTGAAG
TTCAATTGGTACGTGGACGGCGTGGAAGTGCACAACGCCAAGACCAAGCCTAGAGAGGAACAGTAC
AACAGCACCTACAGAGTGGTGTCCGTGCTGACAGTGCTGCATCAGGACTGGCTGAACGGCAAAGAG
TACAAGTGCAAGGTGTCCAACAAGGCCCTGCCTGCTCCTATCGAGAAAACCATCAGCAAGGCCAAGG
GCCAGCCTAGGGAACCCCAGGTTTACACACTGCCTCCAAGCAGGGACGAGCTGACCAAGAATCAGG
TGTCCCTGACCTGCCTCGTGAAGGGCTTCTACCTTCCGATATCGCCGTGGAATGGGAGAGCAATGGC
CAGCCTGAGAACAACTACAAGACAACCCCTCCTGTGCTGGACAGCGACGGCTCATTCTTCCTGTACA
GCAAGCTGACCGTGGACAAGTCCAGATGGCAGCAGGGCAACGTGTTCAGCTGCTCCGTGATGCACG
AGGCCCTGCACAACCACTACACCCAGAAGTCCCTGAGCCTGTCTCCTGGCAAATAA

Based on the positive control antibody HAB20-6-2 prepared above, the recombinant CHO cells could be identified by the flow cytometry. For example, the recombinant CHO cells (CHO-NGF cells) constructed in Example 2 could be identified by the following process:

(1) The CHO-NGF cells constructed in Example 2 and the CHO-S cells used as mock control were cultured in a 37°C, 5% $CO_2$ incubator with the CHOGrow CD1 serum-free medium, and the cell state was adjusted to the logarithmic growth stage.

(2) Each of the above two kinds of cells was added into a well of a 6-well plate in the number of $5\times10^5$ cells, and 100μL of 1×PBS buffer was added to each well with the above cells to resuspend the cells, to each of which 1μg of the positive control antibody HAB20-6-2 was then added. After being mixed thoroughly, they were incubated at room temperature for 45 min; meanwhile, wells without the positive control antibody were set as negative control.

(3) It was centrifuged at room temperature at 800×g for 5 min to remove the supernatant comprising the antibody, and the cells were washed 3 times with 1×PBS buffer.

(4) PE anti-human IgG Fc secondary antibody (dilution of 1:200) was added thereto, which were mixed well and incubated at room temperature away from light for 30 minutes.

(5) It was centrifuged at room temperature at 800×g for 5 min to remove the supernatant comprising the secondary antibody, and the cells were washed 3 times with $1 \times$ PBS buffer.

(6) The cells were resuspended with 500μL of $1 \times$ PBS buffer for flow cytometry assay.

The flow cytometry identification results were shown in Figure 2. According to the flow cytometry detection results of Figure 2, it can be seen that the positivity rate of the positive control antibody against the CHO-NGF cell constructed in Example 2 was 82.67%, and thus it could be used as a target cell for an ADCC effect assay.

**Example 4 Detection of the ADCC effect**

In this example, the CHO-NGF recombinant cell that stably expressed human NGF on the cell surface constructed in the above Example 2 was used as a target cell, and the Jurkat-NFAT-Luciferase-CD16 cell line that stably transfected with luciferase was used as an effector cell. Upon the Fab segment of the sample to be detected or the positive control antibody bound to the antigen on the target cell, the Fc segment of the sample to be detected or the positive control antibody bound to CD16 on the surface of the effector cell Jurkat-NFAT-Luciferase-CD16, and the intracellular NFAT-related signaling pathway was activated, which in turn led to an increase in the luciferase expression level. By detecting changes in luciferase activity, the ADCC effects of the sample to be detected and the positive control antibody were evaluated.

Tanezumab, the sample to be detected, was a recombinant anti-NGF humanized monoclonal antibody developed by Pfizer, which targeted human nerve growth factor and was synthesized by a CHO cell expression system. The relevant information on Tanezumab and the positive control antibody HAB20-6-2 used in this example was shown in the table below.

| Sample Name | Concentration | Storage |
|---|---|---|
| Tanezumab | 10 mg/mL | -70±10°C |
| The positive control antibody HAB20-6-2 | 3.5 mg/mL | -70±10°C |

The ADCC effect assay experiment was conducted using ADCC Reporter Bioassay, and the experimental steps were as follows.

1. Jurkat-NFAT-Luciferase-CD16 overexpression cell was used as an effector cell, and the CHO-NGF cell constructed in Example 2 was used as a target cell. An ADCC experimental system (100μL in total) was established with an effector-target ratio of 1:5, wherein the number of the effector cell was $2\times 10^4$ cells/well, and the number of the target cell was $1 \times 0^5$ cells/well, which were inoculated into a 96-well plate as the subsequent experimental system.

2. Tanezumab and the positive control antibody HAB20-6-2 were firstly diluted with RPMI 1640 medium to a concentration of 200μg/mL to obtain the corresponding stock liquid, respectively, and then 10-fold gradient dilution

was conducted (20μL of each stock liquid obtained by the above dilution was taken and added into 180μL of RPMI 1640 medium, so as to perform the gradient dilution in order), with 9 gradients of continuous dilution (200 μg/mL, 20 μg/mL, 2 μg/mL, 0.2 μg/mL, 0.02 μg/mL, 0.002 μg/mL, 0.0002 μg/mL, 0.00002 μg/mL, 0.000002 μg/mL, and 0 μg/mL).

3. Tanezumab, which was the sample to be detected, and the positive control antibody HAB20-6-2 with different concentration gradients obtained by the above-mentioned dilution were added into the above-mentioned experimental system, respectively, and each well was inoculated with 100μL of the above-mentioned sample to be detected and the positive control antibody (the final concentration thereof in each well was 100 μg/mL, 10 μg/mL, 1 μg/mL, 0.1μg/mL, 0.01μg/mL, 0.001 μg/mL, 0.0001 μg/mL, 0.00001 μg/mL, 0.000001 μg/mL, and 0 μg/mL, respectively), with duplicate wells for each concentration. After being co-cultured in a 37°C, 5% $CO_2$ incubator for 18 hours, 20μL of One-Glo reagent was added thereto, and reacted at room temperature for 5min, and then the ADCC effect of the antibody was evaluated by detecting changes in luciferase activity. Wherein, Tanezumab was repeatedly detected 4 times.

In this example, the CHO-NGF recombinant cell that stably expressed NGF on the cell surface constructed in Example 2 was used as a target cell, and the abilities of the positive control antibody HAB20-6-2 and Tanezumab to mediate the ADCC effect were evaluated by a reporter gene method using the existing effector cell. The detection results of the ADCC effect were shown in Figure 3. According to the detection results in Figure 3, it can be seen that the positive control antibody HAB20-6-2 could cause the Jurkat-NFAT-Luciferase-CD16 effector cell to produce a significant ADCC effect, and there was a significant dose-effect relationship within a certain dose range. However, no significant ADCC effect was detected in four times of detections for Tanezumab that differed from the above positive control antibody only in the constant region sequence of the Fc segment.

It can be seen that under the above experimental conditions, the positive control antibody produced a significant ADCC effect, but Tanezumab, which had the same variable region (from which it could be expected that the antibody Tanezumab could recognize and specifically bind to the same antigen, i.e., the NGF expressed by the CHO-NGF recombinant cell), did not produce a significant ADCC effect. Therefore, the CHO-NGF recombinant cell of the present invention could be effectively used as a target cell for detecting the anti-NGF antibody-mediated ADCC effect *in vitro*, thereby accurately detecting whether the antibody to be detected could cause the effector cell to produce the ADCC effect.

## Example 5 Detection of the CDC effect

In this example, the CHO-NGF recombinant cell that stably expressed human NGF on the cell surface constructed in Example 2 was used as a target cell, and the target cell, human complement serum, and the sample to be detected or the positive control antibody were incubated together. Upon the Fab segment of the sample to be detected or the positive control antibody bound to the antigen on the target cell, the Fc segment of the sample to be detected or the positive control antibody bound to the complement in human serum and resulted in a CDC effect, causing the target cell to be killed. ATP was an important indicator of the metabolism of living cells, and the content thereof directly reflected the number of living cells. Cell Counting-Lite 3D Luminescent reagent was added to the reaction system after completing the incubation. The reagent comprised a thermostable luciferase and the substrate luciferin thereof, and could promote the lysis of living cells in the reaction system to release ATP. ATP promoted the effect of luciferase on the substrate, leading to luminescence of the substrate. By detecting the luminous intensity, the number of living cells in the detection system could be reflected thereby, and the lysis effect of the sample to be detected or the positive control antibody on the target cell via the CDC effect could be further reflected.

The relevant information on Tanezumab, which was the sample to be detected, and the positive control antibody HAB20-6-2 were the same as that in Example 4. The CDC effect detection experiment was conducted according to the following steps:

1. The CHO-NGF cell constructed in Example 2 was used as a target cell, and the cell was inoculated in the amount of $4 \times 10^4$ cells/well (90μL) into the 96-well plate as the subsequent experimental system.

2. Tanezumab, which was the sample to be detected, and the positive control antibody HAB20-6-2 were firstly diluted with RPMI 1640 medium to a concentration of 200 μg/mL to obtain the corresponding stock liquid, respectively, and then 10-fold gradient dilution was conducted (20μL of each stock liquid obtained by the above dilution was taken and added into 180μL of RPMI 1640 medium, so as to perform the gradient dilution in order), with 9 gradients of continuous dilution (200 μg/mL, 20 μg/mL, 2 μg/mL, 0.2 μg/mL, 0.02 μg/mL, 0.002 μg/mL, 0.0002 μg/mL, 0.00002 μg/mL, 0.000002 μg/mL, and 0 μg/mL).

3. Tanezumab, which was the sample to be detected, and the positive control antibody HAB20-6-2 with different concentration gradients obtained by the above-mentioned dilution were added into the above-mentioned experimental system, respectively (the final concentration thereof in each well was 100 μg/mL, 10 μg/mL, 1 μg/mL, 0.1μg/mL, 0.01μg/mL, 0.001 μg/mL, 0.0001 μg/mL, 0.00001 μg/mL, 0.000001 μg/mL, and 0 μg/mL, respectively), and 10μL of Human Complement Serum was added thereto at the same time, with duplicate wells for each concentration. After being co-cultured in a 37°C, 5% $CO_2$ incubator for 18 hours, 50μL of CellCounting-Lite 3D reagent was added thereto, and the cell pellet was oscillated for 2 min for complete lysis; it was placed at room temperature for 25min, followed by detecting the luminescence values using a multifunctional microplate reader. The CDC effect of the antibody was evaluated by detecting changes in the luminescence value to reflect the lysis of the target cell. Wherein, Tanezumab was repeatedly detected 4 times.

4. Calculation formula for the percentage of target cell lysis (Lysis%) ($RLU_{max}$: the fluorescence intensity of the cell mock control wells; $RLU_{sample}$: the fluorescence intensity of the wells with the sample to be detected or the positive control antibody) was as follows:

$$Lysis\% = \left(1 - \frac{RLU_{Sample}}{RLU_{Max}}\right) \times 100\%$$

In this example, the abilities of the positive control antibody HAB20-6-2 and Tanezumab to mediate the CDC effect were evaluated using the CHO-NGF recombinant cell that stably expressed NGF on the cell surface constructed in Example 2 as a target cell. According to the detection results in Figure 4, it can be seen that the positive control antibody HAB20-6-2 could result in a significant CDC effect on the CHO-NGF target cell, and there was a significant dose-effect relationship within a certain dose range. However, no significant CDC effect was detected in the four times detections for Tanezumab that different from the above positive control antibody only in the constant region sequence of the Fc segment.
It can be seen that under the above experimental conditions, the positive control antibody HAB20-6-2 produced a significant CDC effect, but Tanezumab (which had the same variable region as that of the positive control antibody, and it could be expected that the antibody Tanezumab could also recognize and specifically bind to the NGF expressed on the surface of the CHO-NGF recombinant cell) did not produce a significant CDC effect. Therefore, the CHO-NGF recombinant cell of the present invention could be effectively used as a target cell for detecting the anti-NGF antibody-mediated CDC effect *in vitro,* thereby accurately detecting whether the antibody to be detected could produce the CDC effect on the target cell.

**Claims**

1. A recombinant CHO cell, wherein NGF is stably expressed on the surface of the cell.

2. The recombinant CHO cell of claim 1, wherein the NGF is a mammalian NGF, preferably, the NGF is a human NGF, a mouse NGF, a rat NGF, a bovine NGF, a horse NGF or a pig NGF.

3. The recombinant CHO cell of claim 1 or 2, wherein the amino acid sequence of the NGF comprises an amino acid sequence set forth in any one of SEQ ID NO: 1 to SEQ ID NO: 3 or an amino acid sequence having at least 80% sequence identity thereto.

4. The recombinant CHO cell of any of claims 1-3, wherein the genome of the cell is integrated with a nucleic acid sequence of the NGF,
preferably, the nucleic acid sequence of the NGF comprises a nucleic acid sequence set forth in any one of SEQ ID NO: 4 to SEQ ID NO: 6 or a nucleic acid sequence having at least 80% sequence identity thereto.

5. The recombinant CHO cell of any of claims 1-4, wherein the genome of the cell is integrated with a nucleic acid sequence of a concatenated NGF, hinge region and transmembrane region.

6. The recombinant CHO cell of claim 5, wherein the amino acid sequence of the hinge region has 3-100 amino acids, preferably 12-45 amino acids; or
the amino acid sequence of the transmembrane region has 10-60 amino acids, preferably 20-25 amino acids.

7. The recombinant CHO cell of claim 5 or 6, wherein the hinge region is a CD8 hinge region or a human IgG1 hinge region.

8. The recombinant CHO cell of claim 7, wherein the amino acid sequence of the CD8 hinge region comprises an amino acid sequence set forth in SEQ ID NO: 7 or an amino acid sequence having at least 80% sequence identity thereto; or the amino acid sequence of the human IgG1 hinge region comprises an amino acid sequence set forth in SEQ ID NO: 8 or an amino acid sequence having at least 80% sequence identity thereto.

9. The recombinant CHO cell of claim 8, wherein the nucleic acid sequence encoding the CD8 hinge region comprises a nucleic acid sequence set forth in SEQ ID NO: 9 or a nucleic acid sequence having at least 80% sequence identity thereto.

10. The recombinant CHO cell of any of claims 5-9, wherein the transmembrane region is a CD8 transmembrane region, a PGFRA transmembrane region or a CD80 transmembrane region.

11. The recombinant CHO cell of claim 10, wherein the amino acid sequence of the CD8 transmembrane region comprises an amino acid sequence set forth in SEQ ID NO: 10 or an amino acid sequence having at least 80% sequence identity thereto;

the amino acid sequence of the PGFRA transmembrane region comprises an amino acid sequence set forth in SEQ ID NO: 11 or an amino acid sequence having at least 80% sequence identity thereto; or the amino acid sequence of the CD80 transmembrane region comprises an amino acid sequence set forth in SEQ ID NO: 12 or an amino acid sequence having at least 80% sequence identity thereto.

12. The recombinant CHO cell of claim 11, wherein the nucleic acid sequence encoding the CD8 transmembrane region comprises a nucleic acid sequence set forth in SEQ ID NO: 13 or a nucleic acid sequence having at least 80% sequence identity thereto.

13. The recombinant CHO cell of any of claims 5-12, wherein the nucleic acid sequence of the concatenated NGF, hinge region and transmembrane region is a nucleic acid sequence of a concatenated NGF, CD8 hinge region and CD8 transmembrane region.

14. A method of constructing the recombinant CHO cells of any of claims 1-13, comprising:

(1) cloning a nucleic acid sequence of a concatenated NGF, hinge region and transmembrane region into an expression vector to obtain a recombinant expression vector comprising the nucleic acid sequence;
(2) introducing the recombinant expression vector into a CHO cell to obtain the recombinant CHO cell.

15. A system for detecting an ADCC effect or a CDC effect of an antibody, comprising:

(1) the recombinant CHO cell of any of claims 1-13, and

an effector cell;
or (2) the recombinant CHO cell of any of claims 1-13, and
a complement.

16. The system of claim 15, wherein the effector cell is effector T cell, NK cell, phagocyte, eosinophil, basophil or mast cell;

the effector cell is Jurkat-NFAT-Luciferase-CD16 cell or primary NK cell; or
the effector-target ratio of the effector cell to the recombinant CHO cell by number is 1:1 to 1:20.

17. The system of claim 15, wherein the complement is a complement serum comprising complement proteins C1-C9.

18. Use of the recombinant CHO cell of any of claims 1-13 in detecting an ADCC effect or a CDC effect of an antibody.

**Patentansprüche**

1. Rekombinante CHO-(China-Hamster-Ovarial-)Zelle, wobei der NGF auf der Oberfläche der Zelle stabil exprimiert wird.

2. Rekombinante CHO-Zelle nach Anspruch 1, wobei der NGF ein Säugetier-NGF ist, wobei der NGF vorzugsweise ein menschlicher NGF, ein Maus-NGF, ein Ratten-NGF, ein Rinder-NGF, ein Pferde-NGF oder ein Schweine-NGF ist.

3. Rekombinante CHO-Zelle nach Anspruch 1 oder 2, wobei die Aminosäuresequenz des NGF eine Aminosäuresequenz umfasst, die in einer beliebigen der Sequenzen SEQ ID NO: 1 bis SEQ ID NO: 3 festgelegt ist, oder eine Aminosäuresequenz mit mindestens 80 % Sequenzidentität zu dieser.

4. Rekombinante CHO-Zelle nach einem der Ansprüche 1-3, wobei das Genom der Zelle mit einer Nukleinsäuresequenz des NGF integriert ist,
die Nukleinsäuresequenz des NGF vorzugsweise eine Nukleinsäuresequenz umfasst, die in einer beliebigen der Sequenzen SEQ ID NO: 4 bis SEQ ID NO: 6 festgelegt ist, oder eine Nukleinsäuresequenz mit mindestens 80 % Sequenzidentität zu dieser.

5. Rekombinante CHO-Zelle nach einem der Ansprüche 1-4, wobei das Genom der Zelle mit einer Nukleinsäuresequenz aus einem verketteten NGF, einer Scharnierregion und einer Transmembranregion integriert ist.

6. Rekombinante CHO-Zelle nach Anspruch 5, wobei die Aminosäuresequenz der Scharnierregion 3-100 Aminosäuren, vorzugsweise 12-45 Aminosäuren aufweist; oder
die Aminosäuresequenz der Transmembranregion 10-60 Aminosäuren, vorzugsweise 20-25 Aminosäuren aufweist.

7. Rekombinante CHO-Zelle nach Anspruch 5 oder 6, wobei die Scharnierregion eine CD8-Scharnierregion oder eine menschliche IgG1-Scharnierregion ist.

8. Rekombinante CHO-Zelle nach Anspruch 7, wobei die Aminosäuresequenz der CD8-Scharnierregion eine Aminosäuresequenz umfasst, die in Sequenz SEQ ID NO: 7 festgelegt ist, oder eine Aminosäuresequenz mit mindestens 80 % Sequenzidentität zu dieser; oder
die Aminosäuresequenz der menschlichen IgG1-Scharnierregion eine Aminosäuresequenz umfasst, die in Sequenz SEQ ID NO: 8 festgelegt ist, oder eine Aminosäuresequenz mit mindestens 80 % Sequenzidentität zu dieser.

9. Rekombinante CHO-Zelle nach Anspruch 8, wobei die Nukleinsäuresequenz, die die CD8-Scharnierregion kodiert, eine Nukleinsäuresequenz umfasst, die in Sequenz SEQ ID NO: 9 festgelegt ist, oder eine Nukleinsäuresequenz mit mindestens 80 % Sequenzidentität zu dieser.

10. Rekombinante CHO-Zelle nach einem der Ansprüche 5-9, wobei die Transmembranregion eine CD8-Transmembranregion, eine PGFRA-Transmembranregion oder eine CD80-Transmembranregion ist.

11. Rekombinante CHO-Zelle nach Anspruch 10, wobei die Aminosäuresequenz der CD8-Transmembranregion eine Aminosäuresequenz umfasst, die in Sequenz SEQ ID NO: 10 festgelegt ist, oder eine Aminosäuresequenz mit mindestens 80 % Sequenzidentität zu dieser;

die Aminosäuresequenz der PGFRA-Transmembranregion eine Aminosäuresequenz umfasst, die in Sequenz SEQ ID NO: 11 festgelegt ist, oder eine Aminosäuresequenz mit mindestens 80 % Sequenzidentität zu dieser; oder
die Aminosäuresequenz der CD80-Transmembranregion eine Aminosäuresequenz umfasst, die in Sequenz SEQ ID NO: 12 festgelegt ist, oder eine Aminosäuresequenz mit mindestens 80 % Sequenzidentität zu dieser.

12. Rekombinante CHO-Zelle nach Anspruch 11, wobei die Nukleinsäuresequenz, die die CD8-Transmembranregion kodiert, eine Nukleinsäuresequenz umfasst, die in Sequenz SEQ ID NO: 13 festgelegt ist, oder eine Nukleinsäuresequenz mit mindestens 80 % Sequenzidentität zu dieser.

13. Rekombinante CHO-Zelle nach einem der Ansprüche 5-12, wobei die Nukleinsäuresequenz des verketteten NGF, der Scharnierregion und der Transmembranregion eine Nukleinsäuresequenz eines verketteten NGF, einer CD8-Scharnierregion und einer CD8-Transmembranregion ist.

14. Verfahren zum Herstellen der rekombinanten CHO-Zellen nach einem der Ansprüche 1-13, umfassend:

(1) Klonieren einer Nukleinsäuresequenz eines verketteten NGF, einer Scharnierregion und einer Transmembranregion in einen Expressionsvektor, um einen rekombinanten Expressionsvektor zu erhalten, umfassend die Nukleinsäuresequenz;

(2) Einführen des rekombinanten Expressionsvektors in eine CHO-Zelle, um die rekombinante CHO-Zelle zu erhalten.

15. System zum Erfassen eines ADCC-Effekts oder eines CDC-Effekts eines Antikörpers, umfassend:

(1) die rekombinante CHO-Zelle nach einem der Ansprüche 1-13 und

eine Effektorzelle;
oder (2) die rekombinante CHO-Zelle nach einem der Ansprüche 1-13 und
ein Komplement.

16. System nach Anspruch 15, wobei die Effektorzelle eine Effektor-T-Zelle, eine NK-Zelle, ein Phagozyt, ein Eosinophil, ein Basophil oder eine Mastzelle ist;

die Effektorzelle eine Jurkat-NFAT-Luciferase-CD16-Zelle oder eine primäre NK-Zelle ist; oder
das Effektor-Zielverhältnis der Effektorzelle zu der rekombinanten CHO-Zelle in Zahlen 1:1 bis 1:20 beträgt.

17. System nach Anspruch 15, wobei das Komplement ein Komplementserum ist, umfassend die Komplementproteine C1-C9.

18. Verwendung der rekombinanten CHO-Zelle nach einem der Ansprüche 1-13 zum Erfassen eines ADCC-Effekts oder eines CDC-Effekts eines Antikörpers.


**Revendications**

1. Cellule CHO recombinante, dans laquelle le NGF est exprimé de manière stable sur la surface de la cellule.

2. Cellule CHO recombinante selon la revendication 1, dans laquelle le NGF est un NGF de mammifère, de préférence, le NGF est un NGF humain, un NGF de souris, un NGF de rat, un NGF de bovin, un NGF de cheval ou un NGF de cochon.

3. Cellule CHO recombinante selon la revendication 1 ou 2, dans laquelle la séquence d'acides aminés du NGF comprend une séquence d'acides aminés exposée dans l'un quelconque des identificateurs de séquence SEQ ID N°: 1 à SEQ ID N°: 3 ou une séquence d'acides aminés ayant au moins 80 % d'identité de séquence sur celle-ci.

4. Cellule CHO recombinante selon l'une quelconque des revendications 1 à 3, dans laquelle le génome de la cellule est intégré avec une séquence d'acide nucléique du NGF,
de préférence, la séquence d'acide nucléique du NGF comprend une séquence d'acide nucléique exposée dans l'un quelconque des identificateurs de séquence SEQ ID N°: 4 à SEQ ID N°: 6 ou une séquence d'acide nucléique ayant au moins 80 % d'identité de séquence sur celle-ci.

5. Cellule CHO recombinante selon l'une quelconque des revendications 1 à 4, dans laquelle le génome de la cellule est intégré avec une séquence d'acide nucléique d'un NGF concaténé, d'une région charnière et d'une région transmembranaire.

6. Cellule CHO recombinante selon la revendication 5, dans laquelle la séquence d'acides aminés de la région charnière a de 3 à 100 acides aminés, de préférence de 12 à 45 acides aminés ; ou
la séquence d'acides aminés de la région transmembranaire a de 10 à 60 acides aminés, de préférence de 20 à 25 acides aminés.

7. Cellule CHO recombinante selon la revendication 5 ou 6, dans laquelle la région charnière est une région charnière CD8 ou une région charnière IgG1 humaine.

8. Cellule CHO recombinante selon la revendication 7, dans laquelle la séquence d'acides aminés de la région charnière CD8 comprend une séquence d'acides aminés exposée dans l'identificateur de séquence SEQ ID N°:7 ou une séquence d'acides aminés ayant au moins 80 % d'identité de séquence sur celle-ci ; ou
la séquence d'acides aminés de la région charnière IgG1 humaine comprend une séquence d'acides aminés exposée dans l'identificateur de séquence SEQ ID N°: 8 ou une séquence d'acides aminés ayant au moins 80 % d'identité de séquence sur celle-ci.

9. Cellule CHO recombinante selon la revendication 8, dans laquelle la séquence d'acide nucléique codant la région charnière CD8 comprend une séquence d'acide nucléique exposée dans l'identificateur de séquence SEQ ID N°: 9 ou une séquence d'acide nucléique ayant au moins 80 % d'identité de séquence sur celle-ci.

10. Cellule CHO recombinante selon l'une quelconque des revendications 5 à 9, dans laquelle la région transmembranaire est une région transmembranaire CD8, une région transmembranaire PGFRA ou une région transmembranaire CD80.

11. Cellule CHO recombinante selon la revendication 10, dans laquelle la séquence d'acides aminés de la région transmembranaire CD8 comprend une séquence d'acides aminés exposée dans l'identificateur de séquence SEQ ID N°: 10 ou une séquence d'acides aminés ayant au moins 80 % d'identité de séquence sur celle-ci ;

la séquence d'acides aminés de la région transmembranaire PGFRA comprend une séquence d'acides aminés exposée dans l'identificateur de séquence SEQ ID N°: 11 ou une séquence d'acides aminés ayant au moins 80 % d'identité de séquence sur celle-ci ; ou
la séquence d'acides aminés de la région transmembranaire CD80 comprend une séquence d'acides aminés exposée dans l'identificateur de séquence SEQ ID N°: 12 ou une séquence d'acides aminés ayant au moins 80 % d'identité de séquence sur celle-ci.

12. Cellule CHO recombinante selon la revendication 11, dans laquelle la séquence d'acide nucléique codant la région transmembranaire CD8 comprend une séquence d'acide nucléique exposée dans l'identificateur de séquence SEQ ID N°: 13 ou une séquence d'acide nucléique ayant au moins 80 % d'identité de séquence sur celle-ci.

13. Cellule CHO recombinante selon l'une quelconque des revendications 5 à 12, dans laquelle la séquence d'acide nucléique du NGF concaténé, de la région charnière et de la région transmembranaire est une séquence d'acide nucléique d'un NGF concaténé, d'une région charnière CD8 et d'une région transmembranaire CD8.

14. Procédé de construction des cellules CHO recombinantes selon l'une quelconque des revendications 1 à 13, comprenant :

(1) le clonage d'une séquence d'acide nucléique d'un NGF concaténé, d'une région charnière et d'une région transmembranaire en un vecteur d'expression pour obtenir un vecteur d'expression recombinant comprenant la séquence d'acide nucléique ;
(2) l'introduction du vecteur d'expression recombinant dans une cellule CHO pour obtenir la cellule CHO recombinante.

15. Système de détection d'un effet ADCC ou d'un effet CDC d'un anticorps, comprenant :

(1) la cellule CHO recombinante selon l'une quelconque des revendications 1 à 13, et

une cellule effectrice ;
ou (2) la cellule CHO recombinante selon l'une quelconque des revendications 1 à 13, et
un complément.

16. Système selon la revendication 15, dans lequel la cellule effectrice est une cellule T effectrice, une cellule NK, un phagocyte, un éosinophile, un basophile ou un mastocyte ;

la cellule effectrice est une cellule Jurkat-NFAT-Luciferase-CD16 ou une cellule NK primaire ; ou
le rapport effecteur/cible entre la cellule effectrice et la cellule CHO recombinante en nombre est de 1:1 à 1:20.

17. Système selon la revendication 15, dans lequel le complément est un sérum de complément comprenant des protéines de complément C1-C9.

18. Utilisation de la cellule CHO recombinante selon l'une quelconque des revendications 1 à 13 dans la détection d'un effet ADCC ou d'un effet CDC d'un anticorps.

M: Protein Marker

Lane 1: the positive control antibody HAB20-6-2

Figure 1

A

Negative control

The positive control antibody HAB20-6-2

CHO-S cell

R5: 0.110%

R5: 2.000%

B

CHO-NGF cell

R5: 0.920%

R5: 82.670%

Figure 2

Figure 3

Figure 4

## REFERENCES CITED IN THE DESCRIPTION

**Patent documents cited in the description**

- EP 3431494 A1
- EP 0414151 A1
- US 5082774 A
- US 5169762 A
- WO 2021120900 A1
- WO 2021093134 A1
- WO 2021091363 A1
- WO 2019201133 A1
- US 2021079053 A1
- WO 2010128398 A1
- US 2014170137 A1

**Non-patent literature cited in the description**

- **XU L et al.** Expression, purification, and characterization of recombinant mouse nerve growth factor in Chinese hamster ovary cells.. *Protein Expr Purif*, 2014, vol. 104, 41-49
- **WANG XY et al.** Characteristic element of matrix attachment region mediates vector attachment and enhances nerve growth factor expression in Chinese hamster ovary cells.. *Genet Mol Res*, 2015, vol. 14 (3), 9191-9199
- **JIANG JING et al.** Comparison of Recombinant Human β-NGF from E.coli and CHO in Biochemical Characters. *China Biotechnology*, 2006, vol. 26 (2), 8-12
- **SINGLETON et al.** Dictionary of Microbiology and Molecular Biology. J. Wiley & Sons, 1994
- **SAMBROOK et al.** Molecular Cloning, A Laboratory Manual. Cold Springs Harbor Press, 1989
- **DAVIS et al.** Basic Methods in Molecular Biology. Elsevier Science Publishing Inc., 2012
- **ABBAS et al.** Cellular and Molecular Immunology. Elsevier Science Health Science div, 2009
- **HE WEI et al.** Medical Immunology. People's Medical Publishing House Co., Ltd., 2010
- **KATHERINE A. FITZGERALD et al.** The Cytokine FactsBook and Webfacts. Academic Press, 2001
- **WEI MA et al.** , Cloning and Sequencing of Matured Fragment of Human Never Growth Factor Gene. *ACAD J XJ TU*, 2003, vol. 15 (1), 62-65
- **YA BARDE et al.** , The nerve growth factor family. *Progress in Growth Factor Research*, 1990, vol. 2, 237-248
- **WIESMANN, C. et al.** , Nerve growth factor: structure and function. *CMLS, Cell. Mol. Life Sci.*, 2001, vol. 58, 748-759
- **ERIC ADRIAENSSENS et al.** , Nerve Growth Factor Is a Potential Therapeutic Target in Breast Cancer. *Cancer Res.*, 15 January 2008, vol. 68 (2), 346-351
- **LAN RONG et al.** Cell Culture Technology. Chemical Industry Press Co., Ltd., 2007
- **R.I. FERSHNEY**. Culture of Animal Cells: A Manual of Basic Technique and Specialized Applications. John Wiley & Sons, Inc;, 2010
- **LIU BIN et al.** Cell Culture. World Publishing Corporation, 2018